# EUROPEAN PATENT APPLICATION

(11) **EP 3 173 465 A1**
(43) Date of publication of application: **31.05.2017**
(21) Application number: 15196607.4
(22) Date of filing: 26.11.2015
(51) Int. Cl.: C11D 3/20, C12N 9/98, C11D 3/386, C11D 17/00, C11D 17/04

(54) **WATER-SOLUBLE UNIT DOSE ARTICLE**

(71) Applicant: The Procter & Gamble Company, Cincinnati, OH 45202 (US)
(72) Inventor: LANT, Neil Joseph, Newcastle upon Tyne, NE12 9TS (GB); PATTERSON, Steven George, Newcastle upon Tyne, NE12 9TS (GB); NAZARMOHAMMAD, Gulamhussain Momin, Newcastle upon Tyne, NE12 9TS (GB)
(74) Representative: Pickford, James Lawrence

(57) **Abstract**

The present invention is to water-soluble unit dose articles comprising ester-containing laundry ingredients, protease enzymes and lipase enzymes, and their method of use.

## Description

### FIELD OF THE INVENTION

The present invention is to water-soluble unit dose articles comprising ester-containing laundry ingredients, protease enzymes and lipase enzymes, and their method of use.

### BACKGROUND OF THE INVENTION

In the laundry field, water-soluble unit dose articles are preferred by consumers due their efficiency and ease of use. They offer a single dose of detergent which can be dispensed to the washing machine. This is easy and convenient for the consumer and does not result in unnecessary mess from accidental spillages and the like.

Enzymes are preferred cleaning actives of such unit dose articles. Lipase is an effective grease stain removal technology. However, it is difficult to formulate lipase into liquid laundry detergent compositions, especially compacted liquid laundry detergent compositions used in water-soluble unit dose articles, together with protease. This is due to the fact that protease enzymes digest lipases.

A further issue arises when ester-containing laundry ingredients such as polyester soil release polymers are formulated into the liquid laundry detergent compositions. Such polymers are highly preferred as they deliver excellent cleaning benefits. However, they are susceptible to hydrolysis by lipase enzymes.

There remains a need for a water-soluble unit dose article that comprises ester-containing laundry ingredients, especially polyester soil release polymers together with lipase enzymes and protease enzymes.

There also remains a need to provide a water-soluble unit dose article that delivers excellent lipase and protease cleaning benefits yet also further exhibits excellent polyester soil release polymer cleaning benefits in the wash and preferably still has excellent dissolution and release of actives during the wash cycle.

It was surprisingly found that the water-soluble unit dose article of the present invention overcame these technical challenges.

### SUMMARY OF THE INVENTION

A first aspect of the present invention is a water-soluble unit dose article comprising a water-soluble film and at least one internal compartment, wherein the internal compartment comprises a liquid laundry detergent composition, wherein said liquid laundry detergent composition comprises;
a. an ester containing laundry ingredient;
b. a protease enzyme present at a concentration sufficient to provide cleaning benefit to fabrics;
c. a lipase enzyme present at a concentration sufficient to provide cleaning benefits to fabrics;
d. from 5% to 60% by weight of the unit dose article of surfactant;
wherein at least 65% by weight of the lipase enzyme is present in an encapsulate, wherein the encapsulate comprises a shell wherein the shell is insoluble in the liquid laundry detergent composition but which dissolves upon dilution of the liquid laundry detergent composition in the wash liquor.

A second aspect of the present invention is a method of laundering fabrics comprising diluting the water-soluble unit dose article by a factor of at least 500 in water to form a wash liquor and contacting fabrics with said wash liquor.

### DETAILED DESCRIPTION OF THE INVENTION

The present invention is to a water-soluble unit dose article comprising a water-soluble film and at least one internal compartment. The internal compartment comprises a liquid laundry detergent composition.

The liquid laundry detergent composition is described in more detail below.

The water-soluble film is described in more detail below.

The water-soluble unit dose article comprises at least one water-soluble film shaped such that the unit-dose article comprises at least one internal compartment surrounded by the water-soluble film. The at least one compartment comprises the first particle. The water-soluble film is sealed such that the first particle does not leak out of the compartment during storage. However, upon addition of the water-soluble unit dose article to water, the water-soluble film dissolves and releases the contents of the internal compartment into the wash liquor.

The compartment should be understood as meaning a closed internal space within the unit dose article, which holds the particle. Preferably, the unit dose article comprises a water-soluble film. The unit dose article is manufactured such that the water-soluble film completely surrounds the particle and in doing so defines the compartment in which the particle resides. The unit dose article may comprise two films. A first film may be shaped to comprise an open compartment into which the particle is added. A second film is then laid over the first film in such an orientation as to close the opening of the compartment. The first and second films are then sealed together along a seal region. The film is described in more detail below.

The unit dose article may comprise more than one compartment, even at least two compartments, or even at least three compartments. The compartments may be arranged in superposed orientation, i.e. one positioned on top of the other. Alternatively, the compartments may be positioned in a side-by-side orientation, i.e. one orientated next to the other. The compartments may even be orientated in a 'tyre and rim' arrangement, i.e. a first compartment is positioned next to a second compartment, but the first compartment at least partially surrounds the second compartment, but does not completely enclose the second compartment. Alternatively one compartment may be completely enclosed within another compartment.

Wherein the unit dose article comprises at least two compartments, one of the compartments may be smaller than the other compartment. Wherein the unit dose article comprises at least three compartments, two of the compartments may be smaller than the third compartment, and preferably the smaller compartments are superposed on the larger compartment. The superposed compartments preferably are orientated side-by-side.

In a multi-compartment orientation, the first particle according to the present invention may be comprised in at least one of the compartments. It may for example be comprised in just one compartment, or may be comprised in two compartments, or even in three compartments.

Each compartment may comprise the same or different compositions. The different compositions could all be in the same form, or they may be in different forms.

The unit dose article may comprise at least two compartments wherein the liquid laundry detergent composition is comprised within at least one compartment.

The unit dose article may comprise at least two compartments and wherein the liquid laundry detergent composition is comprised within one compartment and a powder composition is comprised within a second compartment.

The water-soluble unit dose article may comprise between 0.5% and 15%, preferably between 1% and 13% by weight of the unit dose article of water.

### Water-soluble film

The film of the present invention is soluble or dispersible in water. The water-soluble film preferably has a thickness of from 20 to 150 micron, preferably 35 to 125 micron, even more preferably 50 to 110 micron, most preferably about 76 micron.

Preferably, the film has a water-solubility of at least 50%, preferably at least 75% or even at least 95%, as measured by the method set out here after using a glass-filter with a maximum pore size of 20 microns:
5 grams ± 0.1 gram of film material is added in a pre-weighed 3L beaker and 2L ± 5ml of distilled water is added. This is stirred vigorously on a magnetic stirrer, Labline model No. 1250 or equivalent and 5 cm magnetic stirrer, set at 600 rpm, for 30 minutes at 30°C. Then, the mixture is filtered through a folded qualitative sintered-glass filter with a pore size as defined above (max. 20 micron). The water is dried off from the collected filtrate by any conventional method, and the weight of the remaining material is determined (which is the dissolved or dispersed fraction). Then, the percentage solubility or dispersability can be calculated.

Preferred film materials are preferably polymeric materials. The film material can, for example, be obtained by casting, blow-moulding, extrusion or blown extrusion of the polymeric material, as known in the art.

Preferred polymers, copolymers or derivatives thereof suitable for use as pouch material are selected from polyvinyl alcohols, polyvinyl pyrrolidone, polyalkylene oxides, acrylamide, acrylic acid, cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides including starch and gelatine, natural gums such as xanthum and carragum. More preferred polymers are selected from polyacrylates and water-soluble acrylate copolymers, methylcellulose, carboxymethylcellulose sodium, dextrin, ethylcellulose, hydroxyethyl cellulose, hydroxypropyl methylcellulose, maltodextrin, polymethacrylates, and most preferably selected from polyvinyl alcohols, polyvinyl alcohol copolymers and hydroxypropyl methyl cellulose (HPMC), and combinations thereof. Preferably, the level of polymer in the pouch material, for example a PVA polymer, is at least 60%. The polymer can have any weight average molecular weight, preferably from about 1000 to 1,000,000, more preferably from about 10,000 to 300,000 yet more preferably from about 20,000 to 150,000.

Mixtures of polymers can also be used as the pouch material. This can be beneficial to control the mechanical and/or dissolution properties of the compartments or pouch, depending on the application thereof and the required needs. Suitable mixtures include for example mixtures wherein one polymer has a higher water-solubility than another polymer, and/or one polymer has a higher mechanical strength than another polymer. Also suitable are mixtures of polymers having different weight average molecular weights, for example a mixture of PVA or a copolymer thereof of a weight average molecular weight of about 10,000- 40,000, preferably around 20,000, and of PVA or copolymer thereof, with a weight average molecular weight of about 100,000 to 300,000, preferably around 150,000. Also suitable herein are polymer blend compositions, for example comprising hydrolytically degradable and water-soluble polymer blends such as polylactide and polyvinyl alcohol, obtained by mixing polylactide and polyvinyl alcohol, typically comprising about 1-35% by weight polylactide and about 65% to 99% by weight polyvinyl alcohol. Preferred for use herein are polymers which are from about 60% to about 98% hydrolysed, preferably about 80% to about 90% hydrolysed, to improve the dissolution characteristics of the material.

Preferred films exhibit good dissolution in cold water, meaning unheated distilled water. Preferably such films exhibit good dissolution at temperatures of 24°C, even more preferably at 10°C. By good dissolution it is meant that the film exhibits water-solubility of at least 50%, preferably at least 75% or even at least 95%, as measured by the method set out here after using a glass-filter with a maximum pore size of 20 microns, described above.

Preferred films are those supplied by Monosol under the trade references M8630, M8900, M8779, M8310.

Of the total PVA resin content in the film described herein, the PVA resin can comprise about 30 to about 85 wt% of the first PVA polymer, or about 45 to about 55 wt% of the first PVA polymer. For example, the PVA resin can contain about 50 w.% of each PVA polymer, wherein the viscosity of the first PVA polymer is about 13 cP and the viscosity of the second PVA polymer is about 23 cP.

Naturally, different film material and/or films of different thickness may be employed in making the compartments of the present invention. A benefit in selecting different films is that the resulting compartments may exhibit different solubility or release characteristics.

The film material herein can also comprise one or more additive ingredients. For example, it can be beneficial to add plasticisers, for example glycerol, ethylene glycol, diethyleneglycol, propylene glycol, sorbitol and mixtures thereof. Other additives may include water and functional detergent additives, including surfactant, to be delivered to the wash water, for example organic polymeric dispersants, etc.

The film may be opaque, transparent or translucent. The film may comprise a printed area. The printed area may cover between 10 and 80% of the surface of the film; or between 10 and 80% of the surface of the film that is in contact with the internal space of the compartment; or between 10 and 80% of the surface of the film and between 10 and 80% of the surface of the compartment.

The area of print may cover an uninterrupted portion of the film or it may cover parts thereof, i.e. comprise smaller areas of print, the sum of which represents between 10 and 80% of the surface of the film or the surface of the film in contact with the internal space of the compartment or both.

The area of print may comprise inks, pigments, dyes, blueing agents or mixtures thereof. The area of print may be opaque, translucent or transparent.

The area of print may comprise a single colour or maybe comprise multiple colours, even three colours. The area of print may comprise white, black, blue, red colours, or a mixture thereof. The print may be present as a layer on the surface of the film or may at least partially penetrate into the film. The film will comprise a first side and a second side. The area of print may be present on either side of the film, or be present on both sides of the film. Alternatively, the area of print may be at least partially comprised within the film itself.

The area of print may comprise an ink, wherein the ink comprises a pigment. The ink for printing onto the film has preferably a desired dispersion grade in water. The ink may be of any color including white, red, and black. The ink may be a water-based ink comprising from 10% to 80% or from 20% to 60% or from 25% to 45% per weight of water. The ink may comprise from 20% to 90% or from 40% to 80% or from 50% to 75% per weight of solid.

The ink may have a viscosity measured at 20°C with a shear rate of 1000s between 1 and 600 cPs or between 50 and 350 cPs or between 100 and 300 cPs or between 150 and 250 cPs. The measurement may be obtained with a cone- plate geometry on a TA instruments AR-550 Rheometer.

The area of print may be achieved using standard techniques, such as flexographic printing or inkjet printing. Preferably, the area of print is achieved via flexographic printing, in which a film is printed, then moulded into the shape of an open compartment. This compartment is then filled with a detergent composition and a second film placed over the compartment and sealed to the first film. The area of print may be on either or both sides of the film.

Alternatively, an ink or pigment may be added during the manufacture of the film such that all or at least part of the film is coloured.

The film may comprise an aversive agent, for example a bittering agent. Suitable bittering agents include, but are not limited to, naringin, sucrose octaacetate, quinine hydrochloride, denatonium benzoate, or mixtures thereof. Any suitable level of aversive agent may be used in the film. Suitable levels include, but are not limited to, 1 to 5000ppm, or even 100 to 2500ppm, or even 250 to 2000rpm.

### Liquid laundry detergent composition

The liquid laundry detergent composition of the present invention comprises;
a. an ester containing laundry ingredient;
b. a protease enzyme present at a concentration sufficient to provide cleaning benefit to fabrics;
c. a lipase enzyme present at a concentration sufficient to provide cleaning benefits to fabrics;
d. from 5% to 60% by weight of the unit dose article of surfactant;
wherein at least 65% by weight of the lipase enzyme is present in an encapsulate, wherein the encapsulate comprises a shell wherein the shell is insoluble in the liquid laundry detergent composition but which dissolves upon dilution of the liquid laundry detergent composition in the wash liquor.

Preferably, the liquid laundry detergent composition has a viscosity of between 300mPa.s and 700mPa.s, more preferably between 350mPa.s and 600mPa.s at a shear rate of 1000s⁻¹. An exemplary method for measuring viscosity is to use a Rheometer DHR1 from TA instruments using a gap of 1000µm at 20°C as according to the manufacturer's instructions.

The liquid laundry detergent composition of the present invention overall is liquid in nature. That is to say, even though it comprises a solid phase dispersed within a liquid phase, the composition has the nature of a liquid rather than a solid or granular composition. In relation to the laundry detergent composition of the present invention, the term 'liquid' encompasses forms such as dispersions, gels, pastes and the like. The liquid composition may also include gases in suitably subdivided form. However, the liquid composition excludes forms which are non-liquid overall, such as tablets or granules.

The term 'liquid laundry detergent composition' refers to any laundry detergent composition comprising a liquid capable of wetting and treating fabric e.g., cleaning clothing in a domestic washing machine,

The water-soluble unit dose article according to claim 1 wherein the liquid laundry detergent composition comprises a structurant, preferably wherein the structurant comprises citrus pulp, hydrogenated castor oils or a mixture thereof.

The ester-containing laundry ingredient is described in more detail below.

The protease enzyme present at a concentration sufficient to provide cleaning benefit to fabrics is described in more detail below.

The lipase enzyme present at a concentration sufficient to provide cleaning benefits to fabrics is described in more detail below.

The surfactant is described in more detail below.

The liquid laundry detergent composition may comprise an adjunct ingredient. Preferably, the adjunct ingredient is selected from bleach, bleach catalyst, dye, hueing dye, cleaning polymers including alkoxylated polyamines and polyethyleneimines, surfactant, solvent, dye transfer inhibitors, encapsulated perfume, cellulosic polymers and mixtures thereof.

### Ester-containing laundry ingredient

The ester-containing laundry ingredient may be any suitable ester-containing material. Ester-containing materials include polymers, surfactants, bleaches, structurants (such as hydrogenated castor oil), perfume materials.

Preferably, the ester-containing laundry ingredient comprises a polyester soil release polymer, preferably wherein the polyester soil release polymer comprises a polypropylene terephthalate.

Suitable polyester soil release polymers may be selected from terephthalate polymers, amine polymers or mixtures thereof. Suitable polyester soil release polymers may have a structure as defined by one of the following structures (I), (II) or (III):

(I) -[(OCHR¹-CHR²)ₐ-O-OC-Ar-CO-]_{d}

(II) -[(OCHR³-CHR⁴)_{b}-O-OC-sAr-CO-]ₑ

(III) -[(OCHR⁵-CHR⁶)_{c}-OR⁷]_{f}

wherein:
a, b and c are from 1 to 200;
d, e and f are from 1 to 50;
Ar is a 1,4-substituted phenylene;
sAr is 1,3-substituted phenylene substituted in position 5 with SO₃Me;
Me is H, Na, Li, K, Mg/2, Ca/2, Al/3, ammonium, mono-, di-, tri-, or tetraalkylammonium wherein the alkyl groups are C₁-C₁₈ alkyl or C₂-C₁₀ hydroxyalkyl, or any mixture thereof; R¹, R², R³, R⁴, R⁵ and R⁶ are independently selected from H or C₁-C₁₈ n- or iso-alkyl; and
R⁷ is a linear or branched C₁-C₁₈ alkyl, or a linear or branched C₂-C₃₀ alkenyl, or a cycloalkyl group with 5 to 9 carbon atoms, or a C₈-C₃₀ aryl group, or a C₆-C₃₀ arylalkyl group. Suitable polyester soil release polymers may be terephthalate polymers having the structure of formula (I) or (II) above.
Suitable polyester soil release polymers include the Repel-o-tex series of polymers such as Repel-o-tex SF2 (Rhodia) and/or the Texcare series of polymers such as Texcare SRA300, or Texcare SRN170 (Clariant).

Other suitable polyester soil release polymers include the polyvinyl ester polymers, preferably graft copolymers of polyvinyl ester. A preferred polymer is a C 1-C6 vinyl ester, preferably polyvinyl acestet grafted onto a polyalkylene oxide. Commercially available polymers of this type include the Sokalan series of materials, for example Sokalan HP-22

Other suitable polymers include the Polyethylene glycol polymers. Polyethylene glycol polymers are those which comprise a polyethylene glycol. Preferably, the polymer backbone comprises the polyethylene glycol and the backbone further comprises side-chains grafted onto said polyethylene glycol backbone. Most preferably, the polyethylene glycol polymer comprises a polyethylene glycol backbone and hydrophobic sidechains. Preferred hydrophobic sidechains are selected from polyvinyl acetate, polyvinyl alcohol and mixtures thereof. Preferably, the polymer comprises from 25% to 60% by weight of the polymer of the backbone.

The perfume may comprise a perfume raw material selected from the group consisting of perfume raw materials having a boiling point (B.P.) lower than about 250°C and a ClogP lower than about 3, perfume raw materials having a B.P. of greater than about 250°C and a ClogP of greater than about 3, perfume raw materials having a B.P. of greater than about 250°C and a ClogP lower than about 3, perfume raw materials having a B.P. lower than about 250°C and a ClogP greater than about 3 and mixtures thereof. Perfume raw materials having a boiling point B.P. lower than about 250°C and a ClogP lower than about 3 are known as Quadrant I perfume raw materials. Quadrant 1 perfume raw materials are preferably limited to less than 30% of the perfume comprosition. Perfume raw materials having a B.P. of greater than about 250°C and a ClogP of greater than about 3 are known as Quadrant IV perfume raw materials, perfume raw materials having a B.P. of greater than about 250°C and a ClogP lower than about 3 are known as Quadrant II perfume raw materials, perfume raw materials having a B.P. lower than about 250°C and a ClogP greater than about 3 are known as a Quadrant III perfume raw materials.

### Protease

The protease is present at a concentration sufficient to provide cleaning benefit to fabrics. A suitable level of protease could be at least 0.001% by weight of the liquid laundry detergent composition.

Suitable proteases include metalloproteases and/or serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:
(a) subtilisins (EC 3.4.21.62), including those derived from *Bacillus*, such as *Bacillus lentus, Bacillus alkalophilus* (P27963, ELYA_BACAO), *Bacillus subtilis*, *Bacillus amyloliqarefaciens* (P00782, SUBT_BACAM), *Bacillus pumilus (P07518) and Bacillus gibsonii* (DSM14391).
(b) trypsin-type or chymotrypsin-type proteases, such as trypsin (e.g. of porcine or bovine origin), including the Fusarium protease and the chymotrypsin proteases derived from *Cellumonas* (A2RQE2).
(c) metalloproteases, including those derived from *Bacillus amyloliqarefaciens* (P06832, NPRE BACAM).

Suitable proteases include those derived from *Bacillus gibsonii* or *Bacillus Lentus* such as subtilisin 309 (P29600) and/or DSM 5483 (P29599).

Suitable commercially available protease enzymes include: those sold under the trade names Alcalase®, Savinase®, Primase®, Durazym®, Polarzyme®, Kannase®, Liquanase®, Liquanase Ultra®, Savinase Ultra®, Ovozyme®, Neutrase®, Everlase® and Esperase® by Novozymes A/S (Denmark); those sold under the tradename Maxatase®, Maxacal®, Maxapem®, Properase®, Purafect®, Purafect Prime®, Purafect Ox®, FN3® , FN4®, Excellase® and Purafect OXP® by Genencor International; those sold under the tradename Opticlean® and Optimase® by Solvay Enzymes; those available from Henkel/Kemira, namely BLAP (P29599 having the following mutations S99D + S101 R + S103A + V104I + G159S), and variants thereof including BLAP R (BLAP with S3T + V4I + V 199M + V205I + L217D), BLAP X (BLAP with S3T + V4I + V205I) and BLAP F49 (BLAP with S3T + V4I + A194P + V199M + V205I + L217D) all from Henkel/Kemira; and KAP (Bacillus alkalophilus subtilisin with mutations A230V + S256G + S259N) from Kao.

The protease enzyme may not be encapsulated.

### Lipase

The lipase is present at a concentration sufficient to provide cleaning benefit to fabrics. A suitable level of lipase could be at least 0.001% by weight of the liquid laundry detergent composition.

Suitable lipases include those of bacterial or fungal origin. Chemically modified or protein engineered mutants are included. Examples of useful lipases include lipases from *Humicola* (synonym *Thermomyces*), e.g., from *H. lanuginosa* (*T. lanarginosars*), or from *H. insolens*, a Pseudomonas lipase, e.g., from *P. alcaligenes* or *P. pseudoalcaligenes, P. cepacia, P. stutzeri*, *P. fluorescens, Pseudomonas* sp. strain SD 705, *P. wisconsinensis, a Bacillus* lipase, e.g., from *B. subtilis, B. stearothermophilus* or *B. pumilus.*

The lipase may be a "first cycle lipase", optionally a variant of the wild-type lipase from *Thermomyces lanuginosus* comprising T231R and N233R mutations. The wild-type sequence is the 269 amino acids (amino acids 23 - 291) of the Swissprot accession number Swiss-Prot 059952 (derived from *Thermomyces lanuginosus* (*Humicola lanuginosa*)). Suitable lipases would include those sold under the tradenames Lipex®, Lipolex® and Lipoclean® by Novozymes, Bagsvaerd, Denmark.

The composition may comprise a variant of *Thermomyces lanuginosa* (059952) lipase having >90% identity with the wild type amino acid and comprising substitution(s) at T231 and/or N233, optionally T231R and/or N233R.

Preferably, the lipase comprises lipase which is a polypeptide having an amino acid sequence which: (a) has at least 90% identity with the wild-type lipase derived from *Humicola lanuginosa* strain DSM 4109; (b) compared to said wild-type lipase, comprises a substitution of an electrically neutral or negatively charged amino acid at the surface of the three-dimensional structure within 15A of E1 or Q249 with a positively charged amino acid; and may further comprise: (I) a peptide addition at the C-terminal; and/or (II) comprises a peptide addition at the N-terminal and/or (III) meets the following limitations: i) comprises a negative amino acid in position E210 of said wild-type lipase; ii) comprises a negatively charged amino acid in the region corresponding to positions 90-101 of said wild-type lipase; and iii) comprises a neutral or negative amino acid at a position corresponding to N94 or said wild-type lipase ; and/or (iv) has a negative or neutral net electric charge in the region corresponding to positions 90-101 of said wild-type lipase; and (v) mixtures thereof.

At least 65%, preferably at least 70%, more preferably at least 80%, most preferably at least 90% by weight of the lipase enzyme is present in an encapsulate.

The shell of the encapsulate preferablycomprises a polymer, copolymer or derivatives thereof, or a mixture thereof, preferably wherein the shell comprises polyvinyl alcohols, polyvinyl pyrrolidone, polyalkylene oxides, acrylamide, acrylic acid, cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides, natural gums, polyacrylates, water-soluble acrylate copolymers, methylcellulose, carboxymethylcellulose, dextrin, ethylcellulose, hydroxyethyl cellulose, hydroxypropyl methylcellulose, maltodextrin, polymethacrylates and combinations thereof.

### Surfactant

The surfactant may comprise an anionic surfactant, a non-ionic surfactant, a cationic surfactant, a zwitterionic surfactant, an amphoteric surfactant or a mixture thereof.

Preferably, the anionic surfactant comprises linear alkylbenzene sulphonate, alkyl sulphate, alkoxylated alkyl sulphate or a mixture thereof.

Exemplary linear alkylbenzene sulphonates are C₁₀-C₁₆ alkyl benzene sulfonic acids, or C₁₁-C₁₄ alkyl benzene sulfonic acids. By 'linear', we herein mean the alkyl group is linear.

The alkoxylated alkyl sulphate anionic surfactant may be a C₁₀-C₁₈ alkyl ethoxy sulfate (AEₓS) wherein x is an average degree of ethoxylation of from 0.5 to 30, preferably between 1 and 10, more preferably between 1 and 5.

Anionic surfactants may comprise fatty acid. The anionic surfactant may comprise linear alkylbenzene sulphonate, alkyl sulphate, alkoxylated alkyl sulphate, fatty acid or a mixture thereof. The term 'fatty acid' includes fatty acid or fatty acid salts. The fatty acids are preferably carboxylic acids which are often with a long unbranched aliphatic tail, which is either saturated or unsaturated. Suitable fatty acids include ethoxylated fatty acids. Suitable fatty acids or salts of the fatty acids for the present invention are preferably sodium salts, preferably C12-C18 saturated and/or unsaturated fatty acids more preferably C12-C14 saturated and/or unsaturated fatty acids and alkali or alkali earth metal carbonates preferably sodium carbonate.

Preferably the fatty acids are selected from the group consisting of lauric acid, myristic acid, palmitic acid, stearic acid, topped palm kernel fatty acid, coconut fatty acid and mixtures thereof.

Preferably, the non-ionic surfactant comprises a fatty alcohol alkoxylate, an oxo-synthesised fatty alcohol alkoxylate, Guerbet alcohol alkoxylates, alkyl phenol alcohol alkoxylates, glycereth cocoate, alkyl polyglucoside or a mixture thereof. The ethoxylated nonionic surfactant may be, e.g., primary and secondary alcohol ethoxylates, especially the C₈-C₂₀ aliphatic alcohols ethoxylated with an average of from 1 to 50 or even 20 moles of ethylene oxide per mole of alcohol, and more especially the C₁₀-C₁₅ primary and secondary aliphatic alcohols ethoxylated with an average of from 1 to 10 moles of ethylene oxide per mole of alcohol.

The ethoxylated alcohol non-ionic surfactant can be, for example, a condensation product of from 3 to 8 mol of ethylene oxide with 1 mol of a primary alcohol having from 9 to 15 carbon atoms.

The non-ionic surfactant may comprise a fatty alcohol ethoxylate of formula R(EO)ₙ, wherein R represents an alkyl chain between 4 and 30 carbon atoms, (EO) represents one unit of ethylene oxide monomer and n has an average value between 0.5 and 20.

### Structurant

The liquid laundry detergent composition may comprise a struacturant. The structurant may be a non-polymeric structurant, preferably a crystallisable glyceride. The structurant may be a polymeric structurant, preferably a fibre based polymeric structurant, more preferably a cellulose fibre-based structurant. The structurant may be selected from crystallisable glyceride, cellulose-fibre based structurants, TiO₂, silica and mixtures thereof.

Suitable structurants are preferably ingredients which impart a sufficient yield stress or low shear viscosity to stabilize the liquid laundry detergent composition independently from, or extrinsic from, any structuring effect of the detersive surfactants of the composition. Preferably, they impart to the laundry detergent composition a high shear viscosity at 20 sec-1 at 21°C of from 1 to 1500 cps and a viscosity at low shear (0.05 sec-1 at 21°C) of greater than 5000 cps. The viscosity is measured using an AR 550 rheometer from TA instruments using a plate steel spindle at 40 mm diameter and a gap size of 500 µm. The high shear viscosity at 20s⁻¹ and low shear viscosity at 0.5s⁻¹ can be obtained from a logarithmic shear rate sweep from 0.1-1 to 25-1 in 3 minutes time at 21°C.

The composition may comprise a non-polymeric crystalline, hydroxyl functional structurant. Such non-polymeric crystalline, hydroxyl functional structurants generally comprise a cystallizable glyceride which can be pre-emulsified to aid dispersion into the final liquid laundry detergent composition. A non-limiting example of such a pre-emulsified external structuring system comprises: (a) crystallizable glyceride(s); (b) anionic surfactant; and (c) water and optionally, non-aminofunctional organic solvents. Each of these components is discussed in detail below.

The structurant may be a polymeric crystalline, hydroxy-functional structurant that comprises a crystallizable glyceride, preferably hydrogenated castor oil or "HCO". HCO as used herein most generally can be any hydrogenated castor oil or derivative thereof, provided that it is capable of crystallizing in the non-polymeric crystalline, hydroxy-functional structurant premix. Castor oils may include glycerides, especially triglycerides, comprising C₁₀ to C₂₂ alkyl or alkenyl moieties which incorporate a hydroxyl group. Hydrogenation of castor oil, to make HCO, converts the double bonds which may be present in the starting oil as ricinoleyl moieties. As such, the ricinoleyl moieties are converted into saturated hydroxyalkyl moieties, e.g., hydroxystearyl. The HCO herein may be selected from: trihydroxystearin; dihydroxystearin; and mixtures thereof. The HCO may be processed in any suitable starting form, including, but not limited to those selected from solid, molten and mixtures thereof. HCO is typically present at a level of from 2% to 10%, from 3% to 8%, or from 4% to 6% by weight in the external structuring system. The corresponding percentage of hydrogenated castor oil delivered into a finished laundry detergent product may be below 1.0%, typically from 0.1% to 0.8%. HCO may be present at a level of between 0.01% and 1%, or even between 0.05% and 0.8% by weight of the laundry detergent composition.

HCO of use in the present invention includes those that are commercially available. Non-limiting examples of commercially available HCO of use in the present invention include: THIXCIN® from Rheox, Inc. Further examples of useful HCO may be found in U.S. Patent 5,340,390.

While the use of hydrogenated castor oil is preferred, any crystallisable glyceride can be used within the scope of the invention. Preferred crystallisable glyceride(s) have a melting point of from 40 °C to 100 °C.

The structurant may comprise a fibre-based structurant. The structurant may comprise a microfibrillated cellulose (MFC), which is a material composed of nanosized cellulose fibrils, typically having a high aspect ratio (ratio of length to cross dimension). Typical lateral dimensions are 1 to 100, or 5 to 20 nanometres, and longitudinal dimension is in a wide range from nanometres to several microns. For improved structuring, the microfibrillated cellulose preferably has an average aspect ratio (l/d) of from 50 to 200,000, more preferably from 100 to 10,000. Microfibrillated cellulose can be derived from any suitable source, including bacterial cellulose, citrus fibers, and vegetables such as sugar beet, chicory root, potato, carrot, and the like.

The structurant may be selected from the group consisting of titanium dioxide, tin dioxide, any forms of modified TiO₂, TiO₂ or stannic oxide, bismuth oxychloride or bismuth oxychloride coated TiO₂, silica coated TiO₂ or metal oxide coated TiO₂ and mixtures thereof. Modified TiO₂ may comprise carbon modified TiO₂, metallic doped TiO₂ or mixtures thereof. Metallic doped TiO₂ may be selected from platinum doped TiO₂, Rhodium doped TiO₂.

The structurant may comprise silica. Those skilled in the art will know suitable silica materials to use. The silica may comprise fumed silica.

### Chelant

Preferably the water-soluble unit dose article comprises a chealnt, preferably wherein the chelant is selected from citrate, 1-hydroxyethane 1,1-diphosphonic acid or a mixture thereof.

The unit dose article may comprise a powder composition wherein the chelant is comprised in the powder composition.

Suitable chelants may be selected from: diethylene triamine pentaacetate, diethylene triamine penta(methyl phosphonic acid), ethylene diamine-N'N'-disuccinic acid, ethylene diamine tetraacetate, ethylene diamine tetra(methylene phosphonic acid), hydroxyethane di(methylene phosphonic acid), and any combination thereof. A suitable chelant is ethylene diamine-N'N'-disuccinic acid (EDDS) and/or hydroxyethane diphosphonic acid (HEDP). The laundry detergent composition may comprise ethylene diamine-N'N'- disuccinic acid or salt thereof. The ethylene diamine-N'N'-disuccinic acid may be in S,S enantiomeric form. The composition may comprise 4,5-dihydroxy-m-benzenedisulfonic acid disodium salt, glutamic acid-N,N-diacetic acid (GLDA) and/or salts thereof, 2-hydroxypyridine-1-oxide, Trilon P^{™} available from BASF, Ludwigshafen, Germany.

Chelants may also act as calcium carbonate crystal growth inhibitors. Suitable calcium carbonate crystal growth inhibitors may be selected from the group consisting of: 1-hydroxyethanediphosphonic acid (HEDP) and salts thereof; N,N-dicarboxymethyl-2-aminopentane-1,5-dioic acid and salts thereof; 2-phosphonobutane-1,2,4-tricarboxylic acid and salts thereof; and any combination thereof.

### Method of making

Those skilled in the art will be aware of how to manufacture a water-soluble unit dose article. An exemplary method is to deform a first water-soluble film into an appropriate mould to form one or more open cavities. The one or more cavities are filled with the first composition and/or second compositions. A second film is then used to close the one or more open cavities.

Those skilled in the art will know how to make the liquid laundry detergent composition using known manufacturing techniques.

### Method of use

The present invention is also to a method of doing laundry comprising the steps of diluting a water-soluble unit dose article according to the present invention in water by a factor of at least 400 to form a wash liquor and then washing fabrics with said wash liquor.

The unit dose article of the present invention may be used alone in the wash operation or may be used in conjunction with other laundry additives such as fabric softeners or fabric stain removers. The unit dose article may be used in conjunction with fragrance boosting compositions such as commercially available 'Lenor Unstoppables'.

The temperature of the wash liquor may be between 10°C and 90°C, preferably between 15°C and 60°C, more preferably between 15°C and 30°C. The wash process may take between 10 minutes and 3.5 hours. The wash process may comprise one or more wash cycles. At least one wash cycle may take between 5 minutes and 2 hours, preferably between 5 minutes and 60 minutes, more preferably between 5 minutes and 40 minutes. The wash process may comprise a combination of short and long cycles. Alternatively, the wash process may comprises a series of short cycles, so-called 'quick wash'. The wash process may be a 'quick wash' at lower temperature.

The articles to be washed may be contacted with the wash liquor or the wash liquor may be contacted with the articles to be washed. Alternatively, the articles to be washed may be present within a washing machine and the wash liquor is formed around them.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A water-soluble unit dose article comprising a water-soluble film and at least one internal compartment, wherein the internal compartment comprises a liquid laundry detergent composition, wherein said liquid laundry detergent composition comprises;
a. an ester containing laundry ingredient;
b. a protease enzyme present at a concentration sufficient to provide cleaning benefit to fabrics;
c. a lipase enzyme present at a concentration sufficient to provide cleaning benefits to fabrics;
d. from 5% to 60% by weight of the unit dose article of surfactant;
wherein at least 65% by weight of the lipase enzyme is present in an encapsulate, wherein the encapsulate comprises a shell wherein the shell is insoluble in the liquid laundry detergent composition but which dissolves upon dilution of the liquid laundry detergent composition in the wash liquor.

2. The water-soluble unit dose article according to claim 1 wherein the liquid laundry detergent composition comprises a structurant, preferably wherein the structurant comprises citrus pulp, hydrogenated castor oils or a mixture thereof.

3. The water-soluble unit dose article according to any preceding claims comprising between 0.5% and 15%, preferably between 1% and 13% by weight of the unit dose article of water.

4. The water-soluble unit dose article according to any preceding claims wherein the ester containing laundry ingredient comprises a polyester soil release polymer, preferably wherein the polyester soil release polymer comprises a polypropylene terephthalate.

5. The water-soluble unit dose article according to any preceding claims wherein the protease enzyme is not encapsulated.

6. The water-soluble unit dose article according to any preceding claims wherein at least 70%, preferably at least 80%, more preferably at least 90% by weight of the lipase enzyme is present in an encapsulate.

7. The water-soluble unit dose article according to any preceding claims wherein the shell of the encapsulate comprises a polymer, copolymer or derivatives thereof, or a mixture thereof, preferably wherein the shell comprises polyvinyl alcohols, polyvinyl pyrrolidone, polyalkylene oxides, acrylamide, acrylic acid, cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides, natural gums, polyacrylates, water-soluble acrylate copolymers, methylcellulose, carboxymethylcellulose, dextrin, ethylcellulose, hydroxyethyl cellulose, hydroxypropyl methylcellulose, maltodextrin, polymethacrylates and combinations thereof.

8. The water-soluble unit dose article according to any preceding claims wherein the lipase comprises wherein the lipase comprises lipase which is a polypeptide having an amino acid sequence which: (a) has at least 90% identity with the wild-type lipase derived from *Humicola lanuginosa* strain DSM 4109; (b) compared to said wild-type lipase, comprises a substitution of an electrically neutral or negatively charged amino acid at the surface of the three-dimensional structure within 15A of E1 or Q249 with a positively charged amino acid; and may further comprise: (I) a peptide addition at the C-terminal; and/or (II) comprises a peptide addition at the N-terminal and/or (III) meets the following limitations: i) comprises a negative amino acid in position E210 of said wild-type lipase; ii) comprises a negatively charged amino acid in the region corresponding to positions 90-101 of said wild-type lipase; and iii) comprises a neutral or negative amino acid at a position corresponding to N94 or said wild-type lipase ; and/or (iv) has a negative or neutral net electric charge in the region corresponding to positions 90-101 of said wild-type lipase; and (v) mixtures thereof.

9. The water-soluble unit dose article according to any preceding claims wherein the water-soluble film comprises polyvinyl alcohol.

10. The water-soluble unit dose article according to any preceding claims wherein the unit dose article comprises at least two compartments wherein the liquid laundry detergent composition is comprised within at least one compartment.

11. The water-soluble unit dose article according to claim 10, wherein the water-soluble unit dose article comprises at least two compartments and wherein the liquid laundry detergent composition is comprised within one compartment and powder composition is comprised within a second compartment.

12. The water-soluble unit dose article according to any preceding claims comprising a chelant, preferably wherein the unit dose article comprises a powder composition and the chelant is comprised in the powder composition.

13. The water-soluble unit dose article according to claim 12 wherein the chelant is selected from citrate, 1-hydroxyethane 1,1-diphosphonic acid or a mixture thereof.

14. A method of laundering fabrics comprising diluting the water-soluble unit dose article by a factor of at least 400 in water to form a wash liquor and washing fabrics with said wash liquor.
